# EUROPEAN PATENT APPLICATION

(11) **EP 2 067 476 A1**
(43) Date of publication of application: **10.06.2009**
(21) Application number: 06775332.7
(22) Date of filing: 09.08.2006
(51) Int. Cl.: A61K 31/704, A61P 1/00, A61P 1/04

(54) **THE APPLICATION OF GLYCYRRHIZIC ACID AND ITS BREAKDOWN PRODUCT GLYCYRRHETINIC ACID FOR THE MANUFACTURE OF A MEDICAMENT FOR THE TREATMENT OF INFLAMMATORY BOWEL DISEASE**

(30) Priority: 17.02.2006 CN 200610018369; 17.02.2006 CN 200610018367
(71) Applicant: Wuhan University, Wuhan City Hubei 430072 (CN); Wuhan Huana Biological Engineering Co. Ltd., Wuhan Hubei 430013 (CN)
(72) Inventor: DING, Hong, Hubei 430013 (CN)
(74) Representative: Redl, Gerda
(86) International application number: PCT/CN2006/002014
(87) International publication number: WO 2007/093090

(57) **Abstract**

The application of glycyrrhizic acid and its breakdown product glycyrrhetinic acid for the manufacture of a medicament for the treatment of inflammatory bowel disease is disclosed. Glycyrrhizic acid or glycyrrhetinic acid and glycyrrhizinate or glycyrrhetate can treat inflammatory bowel disease by inhibiting inflammatory reaction and antioxidation. The medicament prepared by glycyrrhizic acid or glycyrrhetinic acid and glycyrrhizinate or glycyrrhetate has many advantages in treating inflammatory bowel disease, eg., good therapeutic effect and widely safety range.

## Description

### Techniques field:

This discovery is involved with glycyrrhizic acid and its breakdown product glycyrrhetinic acid for the manufacture of a medicament for the treatment of inflammatory bowel disease.

### Background introduction:

Inflammatory bowel disease (IBD) is a kind of persistent disease that remains unclear to doctors and medical researchers. It refers to two chronic diseases that cause inflammatory of the intestines: ulcerative colitis (UC) and Crohn's disease (CD). The former is chronic, nonspecific disease that causes inflammatory in colons. It causes ulcers and even cancers in the serious cases. The primary lesions focus on the colon mucosa and submucosa layers. In clinic, the major symptoms are diarrhea, abdominal pain, and bloody stool with mucus. Different from ulcerative colitis, Crohn's disease can involve any part of the digestive tract, and is a kind of chronic granulomatous inflammation that extends deeper into the layers of the intestinal wall. The major clinical symptoms show diarrhea, abdominal cramping, hemorrhoids and different degrees of symptoms in whole body. In the annual meeting of the tenth year of gastrointestinal society of Europe, Rod Mitchell, the chairman of European Crohn's disease and inflammatory bowel disease, pointed out there were one million of IBD patients in Europe. In western counties, the incidence of ulcerative colitis was stable. The highest record was 10 cases/100,000 people. However, the incidence of Crohn's disease had increased up to 600% for the past 30 years. In our country, it is on the rising trend. This disease can result in higher risk of colon cancer in patients and threatened human health. Although many effects have been made, this kind of disease can not be completely cured.

The common used drugs for IBD treatment include (1) Aminosalicylates: In early 1940's, aminosalicylates had been used for IBD treatment. The better effective drug is combined sulfa drugs arsenic Triprolidine (SASP). However, SASP can cause acute or chronic kidney failures and varying complications, it is not suitable for long term treatment. (2) Corticosteroids: In 1955, Truelove applied corticosteroids to treat UC with excellent feedbacks. So far, corticosteroids still play the major role in IBD treatment. Corticosteroids can effectively control UC in action as well as moderate or severe illness of patients. Because of the potential harmful side effects, it is not suitable for long term treatment. Relapse can happen after stopping drugs uptake. (3) Immunomosuppressants: The application of immunomosuppressants, beginning in 1962. is one of the important methods in IBD therapy. The common drugs include purines, ethotrexate and cyclosporin. Side effects are associated with allergy and inhibit hemocytogenesis. Therefore, to discover a safe and effective new medicine for IBD treatment is critical. Chinese herb medicine has been applied in clinic for thousand years; it provides a abundant drug bank for novel medicine discovery.

The root and stem of licorice (Glycyrrhiza uralensis Fisch) containing glycyrrhizic acid, (or being called Glycyrrhizin), is β-glucuronide of glycyrrhetinic acid. The molecular Formula structure and molecular formula are listed below: 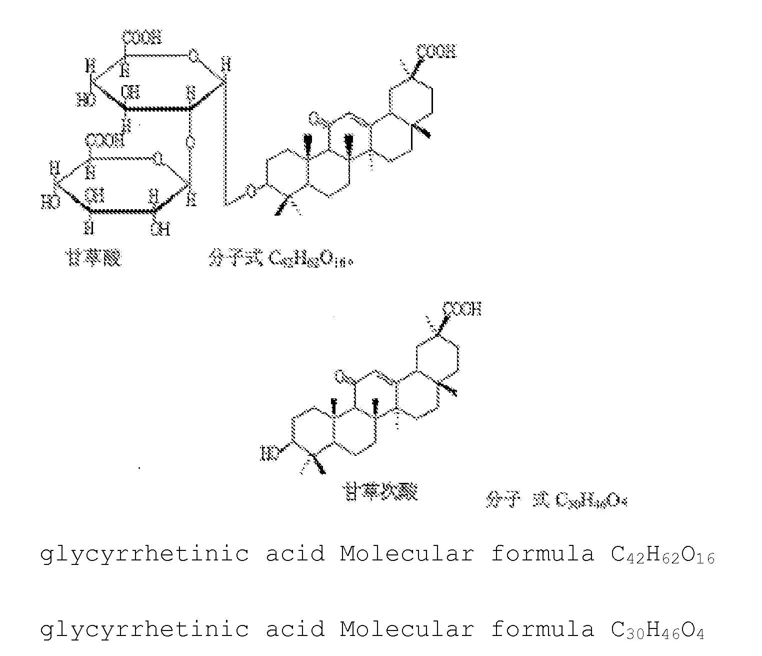 Glycyrrhizic acid and glycyrrhetinic acid have the effects on anti-inflammation, anti-virus, improvement of the detoxification of liver, as well as enhancement of immunity. Furthermore, without having serious side effects as glucocorticoid does, glycyrrhizic acid and glycyrrhetinic acid have been using in the clinical treatment of acute or chronic phase of hepatitis, bronchitis and AIDS diseases. However, it hasn't been reported yet for treatment of anti-inflammation.

### Discovery content

This invention different from current known knowledge and utility referred in the previous paragraph is that this invention discloses glycyrrhizic acid and its breakdown product glycyrrhetinic acid to be used as manufacture of a medicament for the treatment of inflammatory bowel disease.

Strategy I: the application of glycyrrhizin and glycyrrhetinic acid for the treatment of inflammatory bowel disease.

Oral administration of glycyrrhizin 105mg/kg, glycyrrhetinic acid 72mg/kg, once per day for ten days, we observed that CMDI of IBD mice decreased 82.3%, 57.65%; inflammatory scores were lowered 4.0X, 1.72X; MPO activity increased 2.83X, 1.62X.

Injection administration of glycyrrhizin 105mg/kg, glycyrrhetinic acid 72mg/kg, once per day for ten days, we observed that CMDI of IBD mice decreased 98.86%, 63.55%; inflammatory scores were lowered 4.0X, 2.75X; MPO activity increased 3.20X, 2.39X.

In our experiments, we discovered either oral administration or I.P. injection of glycyrrhizin and glycyrrhetinic acid into IBD mice is able to reduce the colon organ injury, CMDI, and MDA obviously, but increase MPO activity, SOD activity, and the amount of GSH significantly. The results demonstrated glycyrrhizin and glycyrrhetinic acid can be used for treatment of IBD.

Strategy II: the application of glycyrrhizinate and glycyrrhetate for the treatment of inflammatory bowel disease

Due to the physical and chemical characteristics of glycyrrhizinate and glycyrrhetate, it is difficult to produce high purity or stable pharmacological reagents. Therefore, in our experiments, glycyrrhizin and glycyrrhetinic acid need to be dissolved in water to in the forms of their salt. The mice in experimental groups are drug-treated by oral administration or intravenous injection once per day. Ten days after the treatment, glycyrrhetate and glycyrrhetinic salts, in the bodies of the mice, were metabolized to glycyrrhizin and glycyrrhetinic acid which can significantly bring down colon organ index, and CMDI; increase colon myeloperoxidase (MPO) activity; lower lipid peroxidase (MDA); enhance superoxide dismutase (SOD) activity; and increase glutathione (GSH) concentration.The results proved that glycyrrhizinate and glycyrrhetate can be used for therapies of inflammatory bowel disease.

### Strategy III:

Oral administration of monoammonium glycyrrhizinate, diammonium glycyrrhizinate, and triammonium glycyrrhizinate with dosage of 14mg/kg, once per day for ten days, we observed that CMDI of IBD mice decreased by 14.3%, 39.4%, 14.9%; inflammatory scores were lowered 1.5X, 1.73X, 1.5X; and MPO activity increased 1.25X, 1.20X, 0.20X.

Diammonium glycyrrhizinate with the dosage of 14mg/kg by injection into mice, once per day for ten days, the results showed that CMDI of IBD mice decreased 45.7%, inflammatory score was lowered 2.75X; and MPO activity increased 1.67X.

In our discovery, glycyrrhizinates include monopotassium glycyrrhizinate, molecular formula: C₄₂H₆₁KO₁₆; dipotassium glycyrrhizinate, molecular formula: C₄₂H₆₀K₂O₁₆; tripotassium glycyrrhizinate, molecular formula: C₄₂H₅₉K₃O₁₆; monosodium glycyrrhizinate, molecular formula: C₄₂H₆₁NaO₁₆; disodium glycyrrhizinate, molecular formula: C₄₂H₆₀Na₂O₁₆; trisodium glycyrrhizinate, molecular formula: C₄₂H₅₉Na₃O₁₆; monoammonium glycyrrhizinate, molecular formula: C₄₂H₆₅NO₁₆; diammonium glycyrrhizinate, molecular formula: C₄₂H₆₈N₂O₁₆; triammonium glycyrrhizinate, molecular formula: C₄₂H₇₁N₃O₁₆; zinc glycyrrhizinate, molecular formula: C₄₂H₆₀ZnO₁₆; magnesium glycyrrhizinate, molecular formula: C₄₂H₆₀MgO₁₆; aluminum glycyrrhizinate, molecular formula: C₄₂H₅₉AlO₁₆.

Likewise, glycyrrhetates in our discovery include monopotassium glycyrrhetate, molecular formula: C₃₀H₄₅KO₄; dipotassium glycyrrhetate, molecular formula: C₃₀H₄₄K₂O₄; tripotassium glycyrrhetate, molecular formula: C₃₀H₄₃K₃O₄; monosodium glycyrrhetate, molecular formula: C₃₀H₄₅NaO₄; disodium glycyrrhetate, molecular formula: C₃₀H₄₂Na₂O₄; trisodium glycyrrhetate, molecular formula: C₃₀H₄₃Na₃O₄; zinc glycyrrhetate, molecular formula: C₃₀H₄₄ZnO₄; magnesium glycyrrhetate, molecular formula: C₃₀H₄₄MgO₄; aluminum glycyrrhetate, molecular formula: C₃₀H₄₃A₁O₄ and Carbenoxolone Sodium, molecular formula: C₃₅H₄₈Na₂O₇.

### Figures discription:

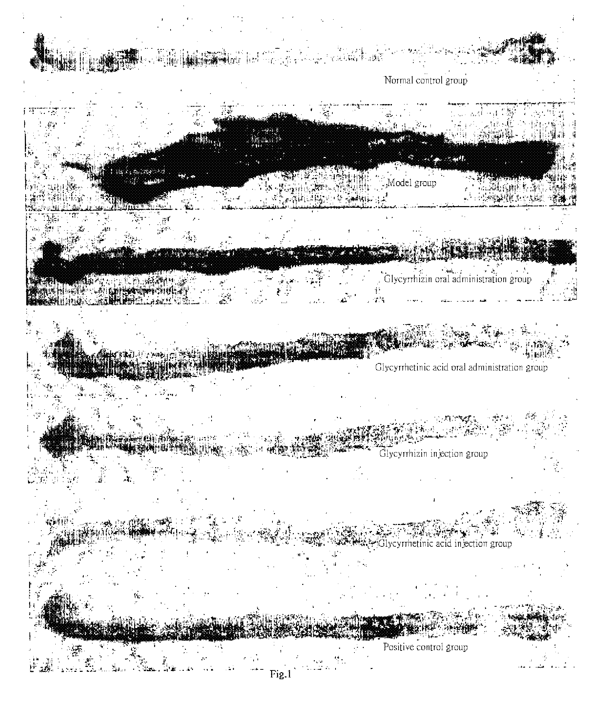

Fig. 1: The protective effects of 10-day treatment of IBD mice with glycyrrhetizin and glycyrrhetic acid on colonic lesion.

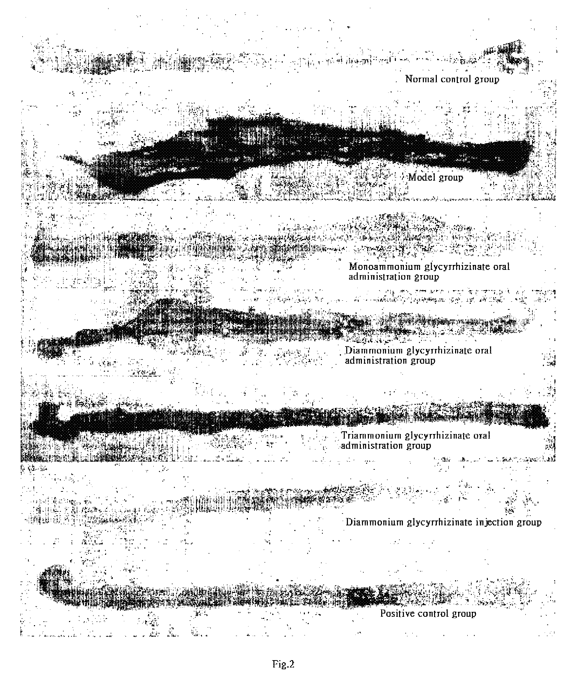

Fig. 2: The effects of 10-day treatment of IBD mice with glycyrrhetate on colonic lesion of IBD mice
Fig. 3: The effects of 10-day drug treatment of TNBS induced IBD rats by disodium glycyrrhetate on colon mucosa (HE x 100)
Fig. 4: The effects of treatment of TNBS induced IBD rats by disodium glycyrrhetate on the surface of colon mucosa (SEM SP x 100)

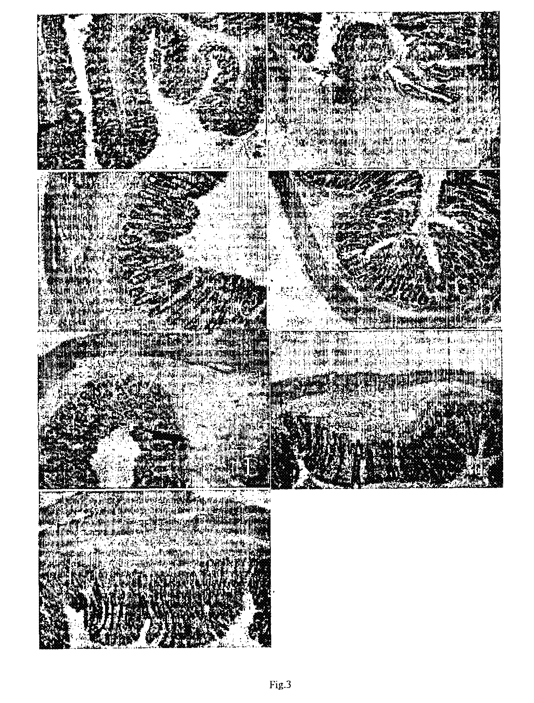

In fig. 3, A: normal control; B: model; C: SASP positive control; D: DG gavage administration group; E: DG low dose group; F: DG medium dose group; G: DG high dose group

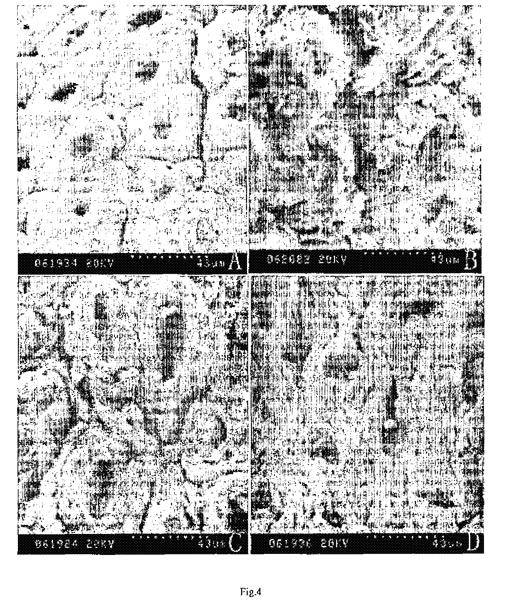

In fig. 4, A: normal control; B: model; C:SASP positive control; D: DG medium dose group

**Experiment I:** Chinese medicines have less side effects for the treatment of chronic or certain other diseases previously referred. In this experiment, we selected glycyrrhizin and glycyrrhetinic acid as anti-IBD drugs. By establishing an IBD animal model, we discovered the effects of glycyrrhizin and glycyrrhetinic acid on IBD and the possible pharmacological mechanisms for disease treatment. The goal is to identify an effective but less toxic anti-IBD drug.

### Material and Method

### Drugs and Reagents

Glycyrrhizic acid, glycyrrhetinic acid provided by SenJiun JiFu Corp.
Adis (slow release granules) provided by the French Apha pharmaceutical company,
MOP kit provided by NanJing JianChen Biology Research Institute
Bovine serum albumin, DTNB, GSH provided by Sigma
TBA, 1.2.3-Benezenetriol, Acetic acid, Ethanol were analytical grade commercial products.

### Animals

45 Healthy mature KuMing mice, weighting range 18 to 22g, half male and half female, provided by Wu Han
University Animal Center (license number: SCXK 2003-2004)
Animal diets were purchased from Wu Han University Animal Center.

### Equipments

TG328A analytical balance, UV spectrophotometer, high-speed homogenizer

### Methods

Kuming mice were randomly divided into groups: normal control, model group, Adis positive control, glycyrrhizic acid oral administration, glycyrrhetinic acid oral administration, glycyrrhizic acid injection, and glycyrrhetinic acid injection group. In oral administration groups, glycyrrhizic acid 105mg/kg and glycyrrhetinic acid 72mg/kg by mouth, once per day. In injection groups, glycyrrhizic acid 105mg/kg and glycyrrhetinic acid 72mg/kg by intraperitoneal injection, once per day. In Adis group, mice were provided Adis 600mg/kg afer enema, once per day. Normal control and model groups were orally given with equal amount of normal saline daily. After seven days drug treatment, all mice except for normal control were stimulated with acetic acid to induce IBD. On day six, starvation was applied after drug treatment to all groups. On day seven, after drug treatment, mice were put to sleep with ether and the procedure of enema was performed before injecting 0.1ml of 6% acitic acid using polyethylene tubings. Acitic acid was injected into mice through anus in depth about 1.5 cm to induce IBD. One minute after the actic acid injection, wash twice with normal saline and let the animals lay down and awake naturally. For normal control, mice were treated with equal amount of saline.

Drug treatment continued to day 10 to all groups. 24 hours after the last drug treatment, the mice were sacrificed. Take and weight the whole colon, score colon organ index, and evaluate CMDI. Colon tissues were homogenized in Tris buffer and adjusted to the concentration 5% for MPO, MDA, SOD, and GSH tests.

### Test indexes

CMDI: Cut open the mouse along the mesentery and take the whole colon. Observe peritoneal adhesion, colon ulcer, and congestion. The standard of evaluation was listed on Table 1.
Myeloperoxidase (MPO): Carefully weight tissue samples, use Tris buffer (weight to volume ratio 1:19) to prepare 5% homogenization solution by high-speed homogenizer. Handle all steps on ice. Protein concentration was determined by method Lowry and use MPO test kit.
Lipid peroxidase (MDA): TBA was used to detect MDA in the homogenization solution
Superoxide dismutas (SOD): Method of pyrogallol was used to detect the activity of SOD in the homogenization solution
Glutathlone (GSH) : Follow the product insert coming with the test kit to detect GSH in the samples

**Table. 1: The standard evaluation of colonic lesion index (CMDI)**

| General characters | Scores |
|---|---|
| **A: Peripheral adhesion** | |
| No adhesion | 0 |
| Slightly adhesion | 1 |
| Severe adhesion | 2 |
| **B: Colon ulcers and inflammation** | |
| Without visible abnormal | 0 |
| Colon congestion; no ulcers | 1 |
| Congestion, no ulcers, but thicken on walls | 2 |
| Ulcer on one spot, no congestion or thicken on walls (necrosis on surface) | 3 |
| Walls with bleeding spots | 4 |
| Ulcer on one spot, congestion, inflammation | 5 |
| Ulcer on two spots, congestion, inflammation | 6 |
| Ulcer on more than two spots | 7 |
| Pierce on wall, one site | 8 |
| Pierce on wall, more than one site | 9 |
| Animals died | 10 |

The total score is combined with both parts of A with B.

### Results:

### 1. The protective effects of glycyrrhizin and glycyrrhetinic acid on the mice with inflammatory bowel disease

The results in table 1 showed that oral administration or injection administration of glycyrrhizin and glycyrrhetinic acid can provide significant protection to the mice with colon injury induced by acetic acid.
In the model group, the mice had severe peripheral adhesion and ulcers on more than two spots. The score was evaluated as 7.5. The positive control was observed with ulcers on one spot, no adhesion, and slight congestion. The score was evaluated as 2.5. The scores for the mice orally given glycyrrhizin and glycyrrhetinic acid were scored as 1.5 and 2.75. As to the mice injected with glycyrrhizin and glycyrrhetinic acid were scored as 1.5 and 2.0. The results showed giving glycyrrhizin and glycyrrhetinic acid orally or by injection can provide significant protection to the mice with colon injury.

### 2. The effects of glycyrrhizin and glycyrrhetinic acid on CMDI, inflammatory score, MPO activity in the IBD mice

The experiment results showed glycyrrhizin and glycyrrhetinic acid can dramatically reduce the colon swelling of IBD mice. Mice oral treated with glycyrrhizin 105mg/kg or glycyrrhetinic acid 72mg/kg, CMDI dropped 82.3%, 57.65%; the inflammatory score lowed 4X, 1.72X; MPO activity rose up 2.83X, 1.62X. Mice injected with glycyrrhizin 105mg/kg or glycyrrhetinic acid 72mg/kg once per day for ten days, CMDI dropped 98.86%, 63.55%; the inflammatory score lowed 4X, 2.75 X; MPO activity rose up 3.20X, 2.39X. Comparing with positive control, the results showed significant effectiveness. (See Table 2)

**Table 2: The effects of glycyrrhizin and glycyrrhetinic acid on CMDI, inflammatory score, MPO activity in the IBD mice**

| (x ± s, n=10) | | | |
|---|---|---|---|
| Groups | CMDI (g/100g) | Inflammatory score | MPO (U/mgpro.) |
| Normal control | 0.73±0.03 | 0±0 | 3.37±0.31 |
| Model | 1.75±0.35** | 7.50±0.7** | 1.33±0.83** |
| Glycyrrhizin (oral treatment) | 0.96±0.03^{ΔΔ} | 1.5±0.31^{ΔΔ} | 5.09±0.29^{ΔΔ} |
| glycyrrhetinic acid (oral treatment) | 1.11±0.41^{ΔΔ} | 2.75±0.78^{ΔΔ} | 3.48±0.46^{ΔΔ} |
| Glycyrrhizin (IP. injection) | 0.88±0.18^{ΔΔ} | 1.5±0.41^{ΔΔ} | 5.59±0.94^{ΔΔ} |
| glycyrrhetinic acid (IP. injection) | 1.07±0.28^{ΔΔ} | 2.0±0.30^{ΔΔ} | 4.51±0.35^{ΔΔ} |
| Positive control | 1.50±0.03^{ΔΔ} | 2.5±0.71^{ΔΔ} | 1.95±0.15^{ΔΔ} |

| | | | |
|---|---|---|---|
| Comparison with normal control ** P<0.01; Comparison with model group ^{ΔΔ} P<0.01 | | | |

### 3. The effects of glycyrrhizin and glycyrrhetinic acid on the concentrations of MDA and GSH, and SOD activity in IBD mice

Compared with the normal control, MDA concentration of colon tissues from the model group is enhanced significantly (P<0.01), GSH and SOD activity dropped. Compared with model group, both glycyrrhizin and glycyrrhetinic acid can improve the above indexes; furthermore, both drugs can provide better protection to IBD mice than positive control. (See Table 3.)

**Table 3. The effects of glycyrrhizin and glycyrrhetinic acid on MDA, GSH concentration and SOD activity in the IBD mice**

| (x ± s, n=10) | | | |
|---|---|---|---|
| Groups | MDA ± (nmol/mgpro.) | GSH (mg/mgpro.) | SOD (U/mgpro.) |
| Normal control | 10.58±2.74 | 0.58±0.12 | 707±105 |
| Model | 26.2±5.5** | 0.16±0.09** | 309±68** |
| Glycyrrhizin (oral treatment) | 6.04±0.89^{ΔΔ} | 0.33±0.22^{ΔΔ} | 425±12^{ΔΔ} |
| glycyrrhetinic acid (oral treatment) | 4.56±2.28^{ΔΔ} | 0.31±0.08^{ΔΔ} | 363±56^{ΔΔ} |
| Glycyrrhizin (IP. injection) | 5.08±1.91^{ΔΔ} | 0.36±0.09^{ΔΔ} | 467±49^{ΔΔ} |
| glycyrrhetinic acid (IP. injection) | 5.85±1.33^{ΔΔ} | 0.31±0.08^{ΔΔ} | 354±58^{ΔΔ} |
| Positive control | 14.29±1.77^{ΔΔ} | 0.37±0.03^{ΔΔ} | 338±15^{Δ} |

| | | | |
|---|---|---|---|
| Comparison with normal control ** P<0.01; Comparison with model group ^{Δ} P<0.05, ^{ΔΔ} P<0.01 | | | |

### Conclusions

Both glycyrrhizin and glycyrrhetinic acid can provide effective protection on IBD mice from colon injury. A possible mechanism may be related to the enhancement of the anti-oxidation activity in tissues. Compared with Adis, a major IBD drug currently available on the market, the efficacy of treatment by glycyrrhizin and glycyrrhetinic acid is better and more beneficial for patients.

**Experiment II:** In this experiment, ammonium glycyrrhizinate were selected as anti-IBD drugs. By establishing an IBD animal model, we demonstrated the effects of ammonium glycyrrhizinate on IBD and the possible pharmacological mechanisms. The goal is to identify an effective but less toxic anti-IBD drugs.

### Material and Method

### Reagents

### Drugs and Reagents

Monoammonium glycyrrhizinate, diammonium glycyrrhizinate, triammonium glycyrrhizinate provided by SenJiun JiFu Corp.
Adis (slow release granules) provided by the French Alpha pharmaceutical company,
MOP kit provided by NanJing JianChen Biology Research Institute
Bovine serum albumin, DTNB, GSH provided by Sigma
TBA, 1.2.3-Benezenetriol, Acetic acid, Ethanol were analytical grade commercial products.

### Animals

45 Healthy mature Kuming mice, weight range from 18 g to 22 g, half male and half female, provided by Wu Han University Animal Center (license number: SCXK 2003-2004)
Animal diets were purchased from Wu Han University Animal Center

### Equipments

TG328A analytical balance, UV spectrophotometer, high-speed homogenizer

### Methods

Kuming mice were randomly distributed into seven groups consisting of normal control, model group, Adis positive control, monoammonium glycyrrhizinate oral administration, diammonium glycyrrhizinate oral administration, triammonium glycyrrhizinate oral administration, diammonium glycyrrhizinate injection.

In Adis group, mice were provided Adis 600mg/kg after enema, once a day. In glycyrrhizinate oral administration groups, 14mg/kg dose of individual glycyrrhizinate was applied once per day. Normal control and model group were orally given by equal amount of normal saline daily. After seven days drug treatment, all mice except for normal control were stimulated with acetic acid to induce IBD. On day six, starvation was applied after drug treatment to all groups. On day seven, after drug treatment, mice were put to sleep with ether and the procedure of enema was performed before injecting 0.1ml of 6% acitic acid using polyethylene tubings. Acitic acid was injected into mice through anus in depth about 1.5 cm to induce IBD. One minute after the actic acid injection, wash twice with normal saline and let the animals lie down and awake naturally. For normal control, mice were treated with equal amount of saline. Drug treatment continued to day 10 to all groups. 24 hours after the last drug treatment, the mice were sacrificed. Take and weight the whole colon, score colon organ index, and evaluate CMDI. Colon tissues were homogenized in Tris buffer and adjusted to the concentration 5% for MPO, MDA, SOD, and GSH tests.

### Test indexes

CMDI: Cut open the mouse along the mesentery and take the whole colon. Observe peritoneal adhesion, colon ulcer, and congestion. The standard of evaluation was listed on Table 1.
Myeloperoxidase (MPO): Carefully weight tissue samples, use Tris buffer (weight to volume ratio 1:19) to prepare 5% homogenization solution by high-speed homogenizer. Handle all steps on ice. Protein concentration was determined by method Lowry and use MPO test kit.

Lipid peroxidase (MDA): TBA was used to detect MDA in the homogenization solution
Superoxide dismutase (SOD): Method of pyrogallol was used to detect the activity of SOD in the homogenization solution
Glutathione (GSH) : Follow the product insert coming with the test kit to detect GSH in the samples

**Table 1: The standard evaluation of colonic lesion index (CMDI)**

| General Characters | Scores |
|---|---|
| **A: Peripheral adhesion** | |
| No adhesion | 0 |
| Slightly adhesion | 1 |
| Severe adhesion | 2 |
| **B: Colon ulcers and inflammation** | |
| Without visible abnormal | 0 |
| Colon congestion; no ulcers | 1 |
| Congestion, no ulcers, but thicken on walls | 2 |
| Ulcer on one spot, no congestion or thicken on walls (necrosis on surface) | 3 |
| Walls with bleeding spots | 4 |
| Ulcer on one spot, 5 congestion, inflammation | |
| Ulcer on two spots, congestion, inflammation | 6 |
| Ulcer on more than two spots | 7 |
| Pierce on wall, one site | 8 |
| Pierce on wall, more than one site | 9 |
| Animals died | 10 |

The total score is combined with both parts of A and B.

### Results:

### 1. The protective effects of glycyrrhizin salts on the mice with inflammatory bowel disease

The result in Table 2 showed that either oral administration or injection of ammonium glycyrrhizinate provided significant protection to the mice with colon injury induced by acetic acid. In the model group, the mice had severe peripheral adhesion and ulcers on more than two spots. The score was evaluated as 7.5. The positive control was observed with ulcers on one spot, no adhesion, and slight congestion. The score was evaluated as 2.5. The mice orally treated with monoammonium glycyrrhizinate, diammonium glycyrrhizinate, triammonium glycyrrhizinate, or injected with diammonium glycyrrhizinate, were scored as 3.0,2.75, 3.0, 2.0. Diammonium glycyrrhizinate injection group had the best protection. The results showed ammonium glycyrrhizinate provided significant protection to IBD. (See Table 2)

### 2. The effects of ammonium glycyrrhizinate on CMDI, inflammatory score, MPO activity in the IBD mice

The results showed ammonium glycyrrhizinate can dramatically reduce the colon swelling of IBD mice. Mice orally treated with monoammonium glycyrrhizinate, diammonium glycyrrhizinate, triammonium glycyrrhizinate, or IP. injected with diammonium glycyrrhizinate, CMDI dropped 14.3%, 39.4%, 14.9%, 45.7%. Comparing with positive control (16.7%), the drug treated groups had better effects. The inflammatory score lowed 1.5X, 1.73 X, 1.5X, 2.75X, similar to the positive control. In addition, The results of MPO activity were rose up 1.25X,, 1.20X, 0.20X, 1.67X, with more significant effects than positive control, 0.47X. These results concluded ammonium glycyrrhizinate benefits to IBD. See Table 2.

**Table 2: The effects of ammonium glycyrrhizinate on CMDI, inflammatory score, MPO activity in the IBD mice**

| (x ± s, n=10) | | | |
|---|---|---|---|
| Groups | CMDI (g/100g) | Inflammatory score | MPO (U/mgpro.) |
| Normal control | 0.73±0.03 | 0±0 | 3.37±0.31 |
| Model | 1.75±0.35** | 7.50±0.7** | 1.33±0.83** |
| monoammonium glycyrrhizinate (oral treatment) | 1.50±0.03^{Δ} | 3.0±0.31^{ΔΔ} | 2.99±0.26^{ΔΔ} |
| diammonium glycyrrhizinate (oral treatment) | 1.06±0.31^{ΔΔ} | 2.75±0.18^{ΔΔ} | 2.93±0.26^{ΔΔ} |
| triammonium glycyrrhizinate (oral treatment) | 1.49±0.08^{ΔΔ} | 3.0±0.21^{ΔΔ} | 1.59±0.94^{ΔΔ} |
| diammonium glycyrrhizinate (injection) | 0.97±0.18^{ΔΔ} | 2.0±0.20^{ΔΔ} | 3.55±0.45^{ΔΔ} |
| Positive control | 1.50±0.03^{ΔΔ} | 2.5±0.71^{ΔΔ} | 1.95±0.15^{ΔΔ} |

| | | | |
|---|---|---|---|
| Comparison with normal control ** P<0.01; Comparison with model group ^{Δ} P<0.05, ^{ΔΔ} P<0.01 | | | |

### 3. The effects of ammonium glycyrrhizinate on MDA and GSH concentrations, and SOD activity in the IBD mice

Compared with normal control, MDA concentration of colon tissues from the model group enhanced significantly (P<0.01), GSH and SOD activity dropped obviously. Compared with model group, ammonium glycyrrhizinate can improve the above indexes; furthermore, work better than positive control. (See Table 3.)

**Table 3. The effects of ammonium glycyrrhizinate on MDA, GSH concentration and SOD activity in the IBD mice**

| (x ± s, n=10) | | | |
|---|---|---|---|
| groups | MDA (nmol/mgpro.) | GSH (mg/mgpro.) | SOD (U/mgpro.) |
| Normal control | 10.58±2.74 | 0.58±0.12 | 707±105 |
| Model | 26.2±5.5** | 0.16±0.09** | 309±68** |
| monoammonium glycyrrhizinate (oral treatment) | 16.14±5.81^{ΔΔ} | 0.32±0.12^{ΔΔ} | 412±112^{ΔΔ} |
| diammonium glycyrrhizinate (oral treatment) | 8.32±5.04^{ΔΔ} | 0.49±0.14^{ΔΔ} | 505±132^{ΔΔ} |
| triammonium glycyrrhizinate (oral treatment) | 16.88±5.97^{ΔΔ} | 0.26±0.09^{ΔΔ} | 368±79^{ΔΔ} |
| diammonium glycyrrhizinate (injection) | 7.85±3.55^{ΔΔ} | 0.27±0.13^{ΔΔ} | 533±101^{ΔΔ} |
| Positive control | 14.29±1.77^{ΔΔ} | 0.37±0.03^{ΔΔ} | 338±15^{Δ} |

| | | | |
|---|---|---|---|
| Comparison with normal control ** P<0.01; Comparison with model group, ^{Δ} P<0.05, ^{ΔΔ} P<0.01 | | | |

### Conclusions

Ammonium glycyrrhizinate can provide effective protection on IBD mice from colon injury. The possible mechanism may be related to the enhancement of the anti-oxidation activity in tissues. Compared with Adis, the major IBD drug on the market, ammonium glycyrrhizinate can provide better and more beneficial treatment. In clinic, ammonium glycyrrhizinate is majorly used as hepatitis treatment. It has wider safe dosage range, low toxic, and neither having side effect like hormones, nor forming the inhibition of immune system like immuolsuppressants. The results showed the dosage range of ammonium glycyrrhizinate being used for IBD treatment falls in the safe range of clinical treatment. Ammonium glycyrrhizinate was approved to be able to provide an effective, safer and less side effect anti-IBD treatment.

**Experiment III:** In this experiment, disodium glycyrrhetate (C₃₀H₄₄Na₂O₄) was selected as anti-IBD drugs. By establishing an IBD animal model, we discovered the effects of disodium glycyrrhetate on IBD and the possible pharmacological mechanisms. The goal is to find out an effective but less toxic anti-IBD drug.

### Materials and Methods

### I:Materials and Equipments

### 1.1 Animals

28 female SPF Wistar rats, weighting range from 230 to 250g, provided by Health and Epidemic Prevention Station of Hubei Province, license number: SCXK 2003-0005, with the feeding conditions of room temperature and 12 hours light period per day.

### 1.2 Drugs and reagents

2,4,6-trinitrobenzene sulfonic acid (TNBS) from Sigma Disodium glycyrrhetate (Lot number: 20060316) from Xian Fouthier Pharmaceutical Co. Ltd.
Sulfasalazine tablets (Lot number: 051001) from Shanghai pharmaceutical Co. Ltd. Cetyltrimethyl bromide (CTAB), Tris-Base, from imported agents
Dianisidine dihydrochloride from Sinopharm Chemical Reagent Co. Ltd
Fecal occult blood test papers (BA-2020B) from Zhuhai flocked (BaSO) Biotechnology Co. Ltd
IL-1β, IL-6 and TNF-α immunoassay kits from Beijing chemclin Biotech Co. Ltd.
Thiobarbituric Acid, hydrochloric acid, Pyrogallol, Disodium hydrogen phosphate, Sodium dihydrogen phosphate, Phosphoric acid, 1-butanol, Ethanol, were analysis-grade reagents

### 1.3 Major equipments:

Inverted microscope (XSP-11CD); UV spectrophotometer (TU-1800PC), TG328A analytical balance, digital pH meter (pHS-25), high speed centrifuge, (Anke TGL-16G), I¹²⁵ radio-isotope counter, S-570 scanning electron microscope (SEM), H-600 transmission electron microscope (TEM), ultra-thin dissector (LKB-V)

### 2. Methods:

### 2.1 Animal grouping

Wister rats were randomly divided into seven groups. There are four mice in each group, including normal control (CON) group, Model (M) group, positive control (SASP) group, diammonium glycyrrhizinate gavage administration (DI) group, diammonium glycyrrhizinate low dose (DGD) group, diammonium glycyrrhizinate medium dose (DGZ) group, and diammonium glycyrrhizinate high dose (DGG) group. No water and diets restriction were applied to mice during the period of the experiment.

### 2.2: TNBS-ethanol induced IBD animal model

Rats were anesthetized by ether. TNBS dissolved in 30% ethanol were injected into rat colon 8 cm in depth through anus with an i.v. tubing. The concentration of TNBS was 100 mg/Kg at the volumes of 0.20 ml/100g. The rats were held up side down for 1 minute after the injection before released back to the cages.

### 2.3: the dose of TNBS-ethanol induced IBD in animal models

There are seven groups total in this test. Rats in all groups were treated identically to create IBD models and the experimental conditions are described in the following.

Normal control (Con) group: Both TNBS-ethanol solution and treated drugs were replaced by normal saline. The dose of drug for treatment was 0.20ml/100g. Model (M) group: After creating the TNBS-ethanol induced IBD model in rats, normal saline instead of drug was used as a blank control for the comparison of drug treatment. The drug dose of IBD treatment was 0.20ml/100g. SASP group: SASP (22.5mg/100g) was used as a positive control. SASP was applied to rats through anus after ether anesthesia. Diammonium glycyrrhetate gavage administration (DI) group: Diammonium glycyrrhetate (10mg/kg) was provided to rats by gavage after ether anesthesia. Diammonium glycyrrhetate, anal administration, low dose (DGD) group: Diammonium glycyrrhetate (2mg/kg) was provided to rats through anus after ether anesthesia. Diammonium glycyrrhetate, anal administration, medium dose (DGZ) group: Diammonium glycyrrhetate (10mg/kg) was provided to rats through anus after ether anesthesia. Diammonium glycyrrhetate, anal administration, high dose (DGG) group: Diammonium glycyrrhetate (50mg/kg) was provided to rats through anus after ether anesthesia.

### 2.4: Test indexes and methods

During the experimental period, daily activities and body weights of rats were investigated and recorded. Ten days after IBD model establishment, fasting was introduced to rats but water was provided continuously. Before sacrificing the rats, feces samples were taken for OB test; eyeballs were removed for blood sampling and serum samples were obtained after centrifugation. Radioimmunoassay kits were used to detect IL-1β, IL-6 and TNF-α, in serum. The rats were cervical executed; organs of spleen and thymus were removed and weighted for the index calculation. The whole length of colon was weighted; weight per unit length of colon was calculated. The degrees of colon injury were evaluated according to the reference 23 with some modifications. We included the changes of intestinal adhesion, colon ulcer, and inflammatory as the score marks. The standard of score is same as we mentioned previously. The 3mm colon samples were taken from the end part of colon; fixed by either neutral formaldehyde solution or 2.5% of glutaraldehyde solution for anatomy dissecting. The samples fixed by neutral formaldehyde solution were sent to perform H-E staining and immunohistochemical staining (IL-10, COX-II, and ICAM-1). The samples fixed by 2.5% of glutaraldehyde solution were photo-imaged by both scanning electron microscope and transmission electron microscope. The rest of samples were handled at ice-cold temperature to make 10% homogenization solution and stored at -20 °C for future use. MOP, SOD, MDA and amounts of tissue proteins were determined by Maehly, Pyrogallol, TBA, and Lowry methods.

### Experiential results

### 1. The effects of Disodium glycyrrhetate on TNBS induced IBD rats, including thymus, spleen, and weight of unit length of colon.

Rats eight days after TNBS induced IBD showed significant difference among the normal control group and the experimental groups. The colonic lesion score of model group showed dramatic difference from control group.
In addition, all drug-treated groups showed significant results in comparison to the model group. Fecal occult blood test was the strongest in the model group than any other groups. DI gavage administration group and DGZ group showed relatively weaker positive results. (See Table 1)

**Table 1. The effects of Disodium glycyrrhetate on colonic lesion score and fecal occult blood test in TNBS induced IBD rats**

| Groups | Dose (mg/100g.) | Colonic lesion score | OB |
|---|---|---|---|
| CON | - | 0 | - |
| M | - | 6.00±3.25** | +++ |
| SASP | 22.5 | 3.00±1.69^{ΔΔ} | ++ |
| DI | 10 | 2.00±1.20^{ΔΔ} | + |
| DGD | 2 | 3.00±1.69^{ΔΔ} | ++ |
| DGZ | 10 | 2.50±1.39^{ΔΔ} | ++ |
| DGG | 50 | 1.50±0.89^{ΔΔ} | + |

| | | | |
|---|---|---|---|
| Comparison with normal control ** P<0.01; Comparison with model group ^{ΔΔ} P<0.01 | | | |

In rats eight days after TNBS induced IBD, the weight of unit length of colon in the model group showed dramatic difference from the control group. In addition, both indexes of spleen and thymus showed significant difference compared to the model group. Drug-treated groups did not show obvious difference on the weight of unit length of colon in comparison to the model group. Except for the DG anal administration, low dose group, the rest experimental groups showed lower weight of unit length of colon than the normal control. DG group worked better than positive control. DGG group got most significant inhibition effect on weight of unit length of colon. (See Table 2)

**Table 2: The effects of Disodium glycyrrhetate on TNBS induced IBD rats, including thymus, spleen, and weight of unit length of colon.**

| groups | Thymus index (mg/g) | Spleen index (mg/g) | Colon weight of length (g/cm) |
|---|---|---|---|
| CON | 0.9302±0.0984 | 2.309±0.135 | 0.106±0.011 |
| M | 0.0319±0.1504** | 3.893±0.210** | 0.376±0.133* |
| SASP | 0.2375±0.0792 | 3.326±0.552 | 0.354±0.087 |
| DI | 0.3161±0.2128 | 3.763±0.780 | 0.240±0.085 |
| DGD | 0.6140±0.3821 | 4.159±0.169 | 0.385±0.218 |
| DGZ | 0.3045±0.2729 | 2.905±0.163^{Δ} | 0.177±0.065 |
| DGG | 0.3647±0.0275 | 3.478±0.687 | 0.174±0.059 |

| | | | |
|---|---|---|---|
| Comparison with normal control ** P<0.01; ** P<0.01 Comparison with model group ^{Δ} P<0.05 | | | |

### 2. The effects of disodium glycyrrhetate on MDA, SOD and SOD in TNBS induced IBD rats.

After investigation of MDA, MPO, and SOD in the homogenized tissue samples, we discovered 1) there is significant difference between MPO in the model group and the normal control; 2) MPO in SASP and DG groups is significant difference in comparison to the model group; 3) MDA and SOD did not show significant difference between the normal control group and the model group, but SOD is lower in the model group than that in the normal control group. In particular, the SOD in the DGG group is significant different than that in the model group. The SOD index from the rest experimental groups did not show significant difference in comparison to the model group. See Table 3 for details.

**Table 3: The effects of disodium glycyrrhetate on MDA, SOD and SOD in TNBS induced IBD rats. (x ± s, n=4).**

| groups | MDA (µmol/mg) | MPO (U/g) | SOD (U/ml) |
|---|---|---|---|
| CON | 0.0360±0.00632 | 21.993±2.061 | 46.400±8.202 |
| M | 0.0274±0.0055 | 134.038±1.979** | 38.005±4.121* |
| SASP | 0.0594±0.0287 | 44.916±3.525^{ΔΔ} | 33.884±2.656 |
| DI | 0.0907±0.0302 | 100.576±10.137^{ΔΔ} | 18.774±8.700 |
| DGD | 0.0431±0.0243 | 141.993±2.494 | 28.847±16.942 |
| DGZ | 0.0365±0.0151 | 83.868±16.465^{Δ} | 46.095±8.065 |
| DGG | 0.0338±0.0115 | 111.753±24.864 | 59.984±3.205^{Δ} |

| | | | |
|---|---|---|---|
| Comparison with normal control * P<0.05; ** P<0.01 Comparison with model group ^{Δ} P<0.05, ^{ΔΔ} P<0.01 | | | |

### 3. The HE staining pictures from colon tissue of rats

In fig. 3, the normal control shows the normal structure of colon tissue comprises by four layers: mucosal layer, submucosa, muscle layer, and serosal layer. The surface of mucosal layer is smooth followed by the lamina propria with large amount of tubular shape, tightly arranged glands. Submucosa contains loosen connective tissue with more lymphoid cells and blood vessels. Muscle layer comprises by the inner ring muscle fibers and the outer longitudinal muscle fibers. Serosal layer sometimes cannot be observed due to unknown factors in the pathological biopsy process. In the model group, typical inflammatory patterns are shown in the mucosal layer. A lot of inflammatory lymphocyte filtration is seen in mucosa, submucosa, and even muscle layers. Ulcers are formed and atypical gland proliferation was also observed. The structures of adjacent mucosal and submucosa layers are distorted; swelling and extension of capillary blood vessels are seen.

In the positive control group, the symptoms are reduced with no ulcers formation. In dissecting slides of colon tissue, the proliferation of mucosa and glands are observed to appear to be normal. The DG gavage administration group showed less severe of symptoms with no ulcer formation. The proliferation of mucosa and glands also seems to be normal.

The DG low dose group still shows relatively severe symptoms but without ulcer formation. Mucosa layers are in the pattern of discontinuous distribution. Inflammatory cell filtration is observed in submucosa and muscle layers. Swelling, congestion and the extension of capillary blood vessels are found in submucosa layer. The DG medium dose group showed relieved symptoms than that of in the DG low dose group; no ulcer formation is seen. Mucosa layers are intact. Inflammatory cell filtration is seen in submucosa and muscle layers. Swelling, congestion and the extension of capillary blood vessels are spotted in submucosa layers. The DG high dose group showed relieved symptoms and no ulcer formation is observed.

### 4. The photo images of colon mucosa tissue of rats (by scanning electron microscope)

In fig 4, the normal control showed that the normal mucosa epithelium with smooth microvillus was divided by grooves to form regular open shaped crypts. Fluid substance is located inside and goblet cells are scattered within epithelium cells. Due to the process of producing biopsy slides, certain surface of colon is deformed. The model group shows typical inflammatory symptoms, including destruction of normal epithelial cell layer, enlargement of gland crests, empty columnar cells, forming irregular volcano like structure. In the positive control group, the symptoms were relieved but with swelling in mucosa layer. Crypts were enlarged; columnar cells were not empty, clearly in recovery stage. DG medium dose group showed relieved symptoms with less swelling. Few crypts were enlarged. Rats were clearly in recovery stage and doing better than positive control.

### Conclusions

TNBS (100mg/kg)-30% ethanol solution can result in serious megacolon formation in treated mice. In disodium glycyrrhetinate high dose group and and gavage administration group, the morphology of rat colon is similar to normal control. The results of smooth inner wall of colon, no visiable congestion, and smaller megacolon formation indicate disodium glycyrrhetinate is able to provide the protective effect on the TNBS induced rat IBD.
In model group, the pathological changes of TNBS-ethanol solution induced typical UC in rats lasted longer. In our research, rats were in acute IBD stage with mucosal necrosis, ulcer formation, and the changes of blood vessels. These changes were caused by ethanol inducing colon tissue necrosis that increased the permeability as well as the distribution of TNBS. The results of H-E staining and EM scanning shown the rats of model group had ulcer formation which was the spontaneous change in mucosal tissues under necrosis.
By edema and inflammatory cells filtration, Blood vessels were squeezed, and the walls of blood vessels were thickened. Some inflammatory substances caused the smooth muscle contraction and worsened local circulation malfunction. All of these factors contributed the lack of blood in local area and increased the range of tissue necrosis. The results of positive control, DG high dose and DG gavage administration groups shown better performance than normal control, with less inflammatory filtration, more normal expression in mucosa, indicated disodium glycyrrhetinate posses the better treatment in TNBS induced IBD rats.

Free radicals play an important role in the pathological process of IBD. Any factors causing massive increase of free radicals in colon tissue eventually result in functional and structural damages in mucosal epithelium cells, as well as activate the functions of colon inflammatory cells and immune cells.
One of the important factors is oxidation free redicals, including superoxide anion radical (O₂-) and hydroxyl radical. Under the strong oxidation reaction, those free radicals can go through lipid peroxidation to generate cell lesions by destroying the structure and function of cell membrane, inactivating cell enzymes, and causing protein denature. The amount of free radicals in TNBS induced IBD rats are higher than in normal control which also related to the level of inflammatory score. Many cases of clinical research demonstrated the level of free radicals in colon mucosa from UC patients significantly increased, anti-oxidation system was defective. The large amount of free radicals can cause cell damage; therefore, anti-oxidation therapy is able to prevent the lesions of colon tissues. Our experiments showed the large amount of free radicals generation in TNBS induced IBD. In DG group, the drug treatment could reduce the content of MDA and MPO activity, but enhanced SOD activity proving the certain mechanism was involved to against inflammatory causing by free radicals, consistent with our findings from the research of glycyrrhetinic acid.

Our experiments demonstrated glycyrrhetinate can relieve inflammatory reaction and reduce colon lesions of TNBS induced IBD. The possible mechanism is involved with anti-oxidation.

## Claims

1. The use of glycyrrhetinic acid and glycyrrhizic acid for manufacture of a medicament for the treatment of inflammatory bowel disease

2. The use of glycyrrhetate for manufacture of a medicament for the treatment of IBD

3. The use according to claim 2, wherein the analogous compounds of glycyrrhetates include monopotassium glycyrrhetate, molecular formula: C₃₀H₄₅KO₄; dipotassium glycyrrhetate, molecular formula: C₃₀H₄₄K₂O₄; tripotassium glycyrrhetate, molecular formula: C₃₀H₄₃K₃O₄; monosodium glycyrrhetate, molecular formula: C₃₀H₄₅NaO₄; disodium glycyrrhetate, molecular formula: C₃₀H₄₂Na₂O₄; trisodium glycyrrhetate, molecular formula: C₃₀H₄₃Na₃O₄; zinc glycyrrhetate, molecular formula: C₃₀H₄₄ZnO₄; magnesium glycyrrhetate, molecular formula: C₃₀H₄₄MgO₄; aluminum glycyrrhetate, molecular formula: C₃₀H₄₃AlO₄ and Carbenoxolone Sodium, molecular formula: C₃₅H₄₈Na₂O₇.

4. The use according to the utilities of the treatment of IBD with glycyrrhizinate, wherein the analogous compounds of glycyrrhizinates include monopotassium glycyrrhizinate, molecular formula: C₄₂H₆₁KO₁₆; dipotassium glycyrrhizinate, molecular formula: C₄₂H₆₀K₂O₁₆; tripotassium glycyrrhizinate, molecular formula: C₄₂H₅₉K₃O₁₆; monosodium glycyrrhizinate, molecular formula: C₄₂H₆₁NaO₁₆; disodium glycyrrhizinate, molecular formula: C₄₂H₆₀Na₂O₁₆; trisodium glycyrrhizinate, molecular formula: C₄₂H₅₉Na₃O₁₆; monoammonium glycyrrhizinate, molecular formula: C₄₂H₆₅NO₁₆; triammonium glycyrrhizinate, molecular formula: C₄₂H₇₁N₃O₁₆; zinc glycyrrhizinate, molecular formula: C₄₂H₆₀ZnO₁₆; magnesium glycyrrhizinate, molecular formula: C₄₂H₆₀MgO₁₆; aluminum glycyrrhizinate, molecular formula: C₄₂H₅₉AlO₁₆.
